(19) Europäisches Patentamt
European Patent Office
Office européen des brevets

(11) Numéro de publication : 0 323 300 B1

(12) # FASCICULE DE BREVET EUROPEEN

(45) Date de publication du fascicule du brevet : 17.04.91 Bulletin 91/16

(51) Int. Cl.$^5$ : C25B 3/04

(21) Numéro de dépôt : 88403171.7

(22) Date de dépôt : 14.12.88

(54) **Procédé de synthèse électrochimique de cétones alpha saturées.**

(30) Priorité : 18.12.87 FR 8717671

(43) Date de publication de la demande : 05.07.89 Bulletin 89/27

(45) Mention de la délivrance du brevet : 17.04.91 Bulletin 91/16

(84) Etats contractants désignés : AT BE CH DE ES FR GB GR IT LI LU NL SE

(56) Documents cités :
FR-A- 2 579 626
FR-A- 2 586 710
CHEMISTRY LETTERS, 1977, pages 1021-1024, Chemical Society of Japan; T. SHONO et al.: "Electroreductive acylation of benzyl chloride and related compounds with acid chlorides"

(73) Titulaire : SOCIETE NATIONALE DES POUDRES ET EXPLOSIFS
12, quai Henri IV
F-75181 Paris Cédex 04 (FR)

(72) Inventeur : Garnier, Laurence
113, boulevard de l'Hopital
F-75013 Paris (FR)
Inventeur : Rollin, Yolande
20 bis, rue de la Procession
F-94470 Boissy Saint Leger (FR)
Inventeur : Perichon, Jacques
20, avenue Saint-Saens
F-91600 Savigny sur Orge (FR)

(74) Mandataire : Pech, Bernard et al
SNPE - Service Propriété Industrielle 12, Quai Henri IV
F-75181 Paris Cédex 04 (FR)

EP 0 323 300 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue Saint-Denis, 75001 PARIS

## Description

L'invention concerne un procédé de synthèse électrochimique de cétones alpha saturées par réduction électrochimique d'halogénures organiques alpha saturés, procédé mis en oeuvre dans une cellule d'électrolyse en milieu solvant organique contenant un électrolyte support.

On entend par cétone alpha saturée une cétone dont les 2 atomes de carbone directement reliés au groupement carbonyle (c'est-à-dire situés en alpha du groupement carbonyle) sont hybridés "$sp_3$" (hybridation tétraédrique). Ces atomes de carbone, encore appelés atomes de carbone "saturés", ne font donc partie ni d'une insaturation éthylénique ou acétylénique, ni d'un cycle ou hétérocycle aromatique.

On entend de même par halogénure organique alpha saturé un halogénure organique dont l'atome de carbone directement relié à l'halogène est hybridé "$sp_3$".

Les cétones et notamment les cétones alpha saturées sont des composés couramment utilisés dans pratiquement tous les domaines de l'industrie chimique, notamment comme solvants ou intermédiaires de synthèse.

SHONO, dans Chemistry Letters, 1977, pages 1021-1024, décrit l'électrosynthèse de cétones benzyliques par réduction électrochimique de chlorures benzyliques en présence de chlorures d'acides carboxyliques en milieu acétonitrile ou N,N-diméthylformamide (DMF). La cellule comprend nécessairement deux compartiments séparés par un diaphragme en céramique et l'anode est en carbone. Les rendements chimique et faradique sont assez faibles, surtout dans le DMF.

FR 2 579 626, dont la Demanderesse est titulaire, propose un procédé plus simple et procurant des rendements supérieurs. On parvient à ce résultat par réduction électronique d'hologénures en utilisant un anhydride d'acide organique comme dérivé d'acide organique et une anode en un métal choisi dans le groupe constitué par le magnésium, le zinc, l'aluminium et leurs alliages.

La manipulation d'anhydrides est toutefois souvent délicate.

La présente invention propose un procédé de synthèse électrochimique de cétones alpha saturées tout aussi simple que celui décrit dans FR 2 579 626 mais mis en oeuvre à partir de $CO_2$ et d'halogénures organiques alpha saturés. L'utilisation de $CO_2$, gaz courant, bon marché, facile à manipuler à la place d'anhydrides d'acides ou de chlorures d'acides dans les procédés connus, présente un intérêt pratique et économique certain.

Le procédé selon l'invention d'électrosynthèse de cétones alpha saturées par réduction électrochimique d'halogénures organiques alpha saturés dans une cellule d'électrolyse munie d'électrodes en milieu solvant organique contenant un électrolyte support, l'anode étant en un métal choisi dans le groupe constitué par les métaux réducteurs et leurs alliages, caractérisé ce que la réduction a lieu en présence de $CO_2$ et d'un catalyseur à base de nickel complexé par un ligand organique bidentate biazoté. On entend par "leurs alliages" tout alliage contenant au moins un métal réducteur.

De façon préférée, l'anode est en un métal choisi dans le groupe constitué par le zinc, l'aluminium, le magnésium et leurs alliages, à savoir tout alliage contenant au moins du zinc, de l'aluminium ou du magnésium. Elle est consommée au cours de la réduction électrochimique dont elle est le siège. Il s'agit donc d'un procédé à anode consommable encore appelé "à anode soluble". De façon particulièrement préférée l'anode est en un métal choisi dans le groupe constitué par le magnésium et ses alliages, à savoir les alliages contenant du magnésium.

De façon préférée les halogénures organiques alpha saturés répondent à la formule générale (A) :

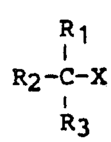

dans laquelle

X représente un atome d'halogène, de préférence le chlore ou le brome

•$R_1$, $R_2$ et $R_3$, identiques ou différents, représentent un atome d'hydrogène ou un radical organique, aliphatique, aromatique ou hétérocyclique, saturé ou insaturé, substitué ou non substitué, éventuellement substitué par au moins un groupement non électroréductible dans les conditions de l'électrolyse, comme par exemple un groupement éther, ester ou nitrile,

ou bien encore $R_1$ et $R_2$ forment un cycle aliphatique.

De façon particulièrement préférée, $R_1$ et $R_2$ représentent l'hydrogène et $R_3$ représente soit un groupement hydrocarboné saturé ou insaturé, substitué ou non substitué, soit un groupement aromatique, tel qu'un groupement phényle éventuellement substitué.

Comme exemple de groupements hydrocarbonés saturés on peut citer les chaînes alkyles ayant un nombre d'atomes de carbone inférieur ou égal à 15 et comme exemple de groupements hydrocarbonés insaturés les groupements vinyliques.

Les halogénures organiques préférés selon l'invention sont les bromures d'alknyle de formule générale $C_nH_{2n+1}$ Br ($n \leq 15$) ainsi que les chlorure ou bromure benzyliques et les chlorure ou bromure allyliques.

Selon une variante, on réduit un seul halogénure organique alpha saturé. Si on représente cet halogènure par la formule RX, on obtient la cétone symétrique de formule

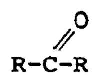

Selon une autre variante de l'invention on réalise la réduction électrochimique d'un mélange de plusieurs halogénures organiques alpha saturés différents. On obtient ainsi un mélange de cétones mixtes et de cétones symétriques.

Selon cette variante, on utilise de préférence un mélange de 2 halogénures organiques alpha saturés différents. Si on représente ces 2 halogénures par les formules RX et R'X', on obtient un mélange des 3 cétones suivantes :

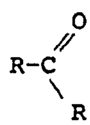 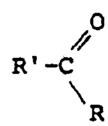 et 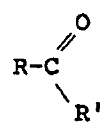

Le catalyseur à base de nickel est obtenu par mélange d'un halogénure de nickel, de préférence $NiBr_2$, et d'un ligand organique bidentate biazoté. On utilise de préférence un excès de ligand, par exemple 1 à 10 moles de ligand par mole d'halogénure de nickel.

Le ligand organique bidentate biazoté est de préférence la 2,2'bipyridine. Dans ce cas on utilise de préférence 2 à 3 moles de ligand par mole d'halogénure de nickel.

De façon inattendue, on a constaté qu'on obtient des rendements nettement supérieurs lorsque le catalyseur est obtenu en présence d'un coligand éthylénique.

On obtient par exemple un tel catalyseur en utilisant la 2,2'bipyridine à raison de 1 à 1,5 moles par mole d'halogénure de nickel et en maintenant une pression partielle d'éthylène sur la solution.

La concentration en catalyseur dans le solvant organique est en général comprise entre $10^{-3}$ et 5 $10^{-2}$ M, de préférence entre $10^{-2}$ et 2 $10^{-2}$ M.

L'halogénure organique alpha saturé, ou le mélange d'halogénures organiques alpha saturés, est de préférence ajouté progressivement au cours de la réaction, de façon à maintenir une concentration dans le solvant organique proche de celle du catalyseur.

On obtient ainsi, de façon inattendue, de meilleurs rendements. La quantité totale d'halogénure ajouté peut atteindre une mole par litre de solvant, de préférence 0,2 à 0,5 mole.

La pression en $CO_2$ est en général comprise entre 0,01 MPa et 1 MPa. On travaille de préférence à la pression atmosphérique (environ 0,1 MPa).

La cathode est un métal quelconque tel que l'acier inoxydable, le nickel, le platine, le cuivre, l'or, ou du graphite. Elle est constituée, de façon préférée, par une grille ou une plaque cylindrique disposée concentriquement autour de l'anode.

Les électrodes sont alimentées en courant continu par l'intermédiaire d'une alimentation stabilisée.

Les solvants organiques utilisés dans le cadre de la présente invention sont les solvants peu pratiques utilisés usuellement en électrochimie organique. On peut citer par exemple le DMF, l'acétonitrile, la tétraméthylurée (TMU), la N-méthylpyrrolidone (NMP), l'hexaméthylphosphorotriamide (HMPT) et les mélanges de ces produits.

On utilise de préférence le DMF ou la NMP.

Les électrolytes support utilisés pour rendre le milieu conducteur sont ceux habituellement utilisés en électrochimie organique. On peut citer par exemple les sels dont l'anion est un halogénure, un perchlorate ou un fluoroborate et le cation un ammonium quaternaire, le lithium, le sodium, le potassium, le magnésium, le zinc ou l'aluminium.

On utilise de façon préférée le fluoroborate de tétrabutylammonium ou le bromure de tétrabutylammonium.

De façon préférée, la concentration en électrolyte support dans le solvant organique est comprise entre 0,01 M et 0,5 M.

La densité de courant sur la cathode est de préférence choisie entre 0,2 et 5 $A/dm^2$. On opère en général à intensité constante, mais on peut également opérer à tension constante, à potentiel contrôlé ou avec intensité et potentiel variables.

L'invention est illustrée par les exemples non limitatifs qui vont suivre. Pour réaliser ces exemples, on utilise une cellule classique non compartimentée.

La partie supérieure de la cellule est en verre et est équipée de 5 tubulures qui permettent l'arrivée et la sortie de gaz, les prélèvements éventuels de solution en cours d'électrolyse, les passages électriques et le passage de l'anode par une tubulure centrale.

La partie inférieure est constituée par un bouchon muni d'un joint, vissé sur la partie supérieure en verre.

Le volume utile est d'environ 35 $cm^3$.

L'anode est un barreau cylindrique de 0,5 cm de diamètre qui plonge dans la solution sur une longueur d'environ 2 cm. Elle se trouve en position axiale par rapport à la cellule.

La cathode est constituée par une grille disposée concentriquement autour de l'anode. La surface de travail de la cathode est de l'ordre de 10 $cm^2$.

Le solvant est purifié par distillation sous vide.

La solution est agitée par l'intermédiaire d'un barreau aimanté et l'électrolyse a lieu à température ambiante.

Les produits obtenus sont dosés, isolés, purifiés et identifiés selon des méthodes classiques.

Exemple 1 – Synthèse de di n-hexylcétone

La cathode est en or et l'anode en magnésium.

On introduit dans la cellule 30 $cm^3$ de NMP, 3 mmol de fluoroborate de tétrabutylammonium, 0,6 mmol de $NiBr_2$, et 1,5 mmol de 2,2'bipyridine.

On maintient la cellule sous atmosphère de $CO_2$, en légère surpression par rapport à la pression atmosphérique.

On électrolyse ce mélange durant 40 min sous un courant d'intensité constante de 0,05 A puis, tout en poursuivant l'électrolyse, on ajoute de manière continue 12 mmol de bromure de n-hexyle à raison de 0,9 mmol par heure.

Quand tout le bromure de n-hexyle est ajouté on arrête l'électrolyse. On hydrolyse ensuite la solution avec une solution aqueuse d'acide chlorhydrique 1 M puis on effectue une extraction au pentane.

La phase organique, après évaporation des solvants, fournit un produit brut dont l'analyse par chromatographie en phase gazeuse (CPG) indique qu'on a obtenu la dihexylcétone avec un rendement de 70% par rapport au bromure de n-hexyle utilisé. Le produit est purifié et isolé par chromatographie sur colonne de silice et identifié par ses spectres IR, RMN et de masse.

Exemple 2 – Synthèse de di n-hexylcétone.

On opère comme pour l'exemple 1 mais on n'utilise que 0,7 mmol de 2,2'-bipyridine et on remplace le $CO_2$ par un mélange 50/50 en volumes de $CO_2$ et d'éthylène.

Le rendement en dihexylcétone obtenue est de 90%.

Exemples 3 à 7 – Synthèse de diverses cétones alpha saturées.

On opère comme pour l'exemple 1 mais avec d'autres halogénures organiques alpha saturés dont la vitesse d'addition est de 1,8 mmol/h au lieu de 0,9 mmol/h.

Le tableau 1 suivant précise l'halogénure utilisé ainsi que le rendement et la nature de la cétone obtenue.

TABLEAU 1

| EXEMPLE N° | HALOGENURE ORGANIQUE | CETONE OBTENUE | RENDEMENT (%) |
| --- | --- | --- | --- |
| 3 | $\phi$ $CH_2Br$ | $(\phi CH_2)_2CO$ | 85 |
| 4 | $\phi$ $CH_2Cl$ | $(\phi CH_2)_2CO$ | 70 |
| 5 | $CH_3-CH=CH-CH_2Br$ | $(CH_3-CH=CH-CH_2)_2CO$ | 70 |
| 6 | $CH_3-CH=CH-CH_2Cl$ | $(CH_3-CH=CH-CH_2)_2CO$ | 40 |
| 7 | 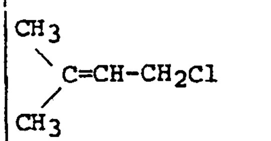 | 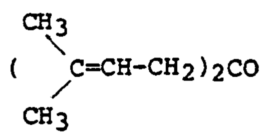 | 30 |

$\phi$ signifie $C_6H_5$.

Exemples 8 à 12 – Synthèse de dibenzylcétone.

On opère comme pour les exemples 3 et 4 mais avec modification du solvant ou de l'électrolyte support.

Dans tous ces exemples on obtient la dibenzylcétone. Le tableau 2 suivant précise l'halogénure organique de départ, le solvant utilisé, la nature et la quantité d'électrolyte support ainsi que le rendement en dibenzylcétone obtenue, exprimé par rapport à l'halogénure organique de départ.

TABLEAU 2

| EXEMPLE N° | HALOGENURE ORGANIQUE | SOLVANT | ELECTROLYTE SUPPORT | RENDEMENT (%) |
|---|---|---|---|---|
| 8 | $\phi$ $CH_2Br$ | DMF | $NBu_4BF_4$ (3mmol) | 33 |
| 9 | $\phi$ $CH_2Br$ | DMF | $NBu_4Br$ (3mmol) | 77 |
| 10 | $\phi$ $CH_2Cl$ | NMP | $NBu_4Br$ (3mmol) | 77 |
| 11 | $\phi$ $CH_2Cl$ | NMP | $NBu_4Br$(1,5mmol) | 40 |
| 12 | $\phi$ $CH_2Br$ | DMF | $NBu_4Br$ (6mmol) | 80 |

$\phi$ signifie $C_6H_5$.

Exemple 13 – Synthèse de dibenzylcétone.

On opère comme pour l'exemple 3 mais on utilise une cathode en nickel doré. Le rendement est de 76%.

Exemple 14 – Synthèse de dibenzylcétone.

On opère comme pour l'exemple 9 mais on utilise une cathode en carbone vitreux. Le rendement est de 77%.

Exemples 15 à 18 – Synthèse de divers cétones alpha saturées.

On opère dans les mêmes conditions que celles de l'exemple 1 avec d'autres halogénures alpha saturés mais avec seulement 0,3 mmol de $NiBr_2$ et 0,75 mmol de 2,2'-bipyridine.

Le tableau 3 suivant précise pour chaque exemple l'halogénure de départ, la nature et le rendement, par rapport à l'halogénure de départ, de la cétone obtenue.

TABLEAU 3

| EXEMPLE N° | HALOGENURE ORGANIQUE | CETONE OBTENUE | RENDEMENT (%) |
|---|---|---|---|
| 15 | $nC_7H_{15}Br$ | $(nC_7H_{15})_2CO$ | 70 |
| 16 | $\Phi CH_2Cl$ | $(\Phi CH_2)_2CO$ | 70 |
| 17 | $CH_3CH=CH-CH_2Br$ | $(CH_3CH=CH-CH_2)_2CO$ | 80 |
| 18 | 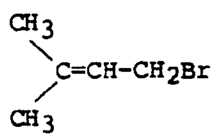 | 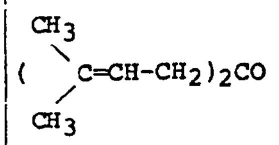 | 50 |

$\Phi$ signifie $C_6H_5$

Exemple 19 – Synthèse de benzyl n-hexylcétone.

On opère dans les mêmes conditions que celles de l'exemple 3 mais on remplace le bromure de benzyle par un mélange équimolaire de chlorure de benzyle et de bromure de n-hexyle. On obtient la cétone $nC_6H_{13}COCH_2\phi$ avec un rendement de 25%.

## Revendications

1. Procédé de synthèse électrochimique de cétones alpha saturées par réduction électrochimique d'halogénures organiques alpha saturés dans une cellule d'électrolyse munie d'électrodes en milieu solvant organique contenant un électrolyte support, l'anode étant en un métal choisi dans le groupe constitué par les métaux réducteurs et leurs alliages caractérisé en ce que la réduction a lieu en présence de $CO_2$ et d'un catalyseur à base de nickel complexé par un ligand organique bidentate biazoté.

2. Procédé selon la revendication 1 caractérisé en ce que l'anode est en un métal choisi dans le groupe constitué par le magnésium et ses alliages.

3. Procédé selon l'une quelconque des revendications précédentes caractérisé en ce que les halogénures organiques alpha saturés répondent à la formule générale (A)

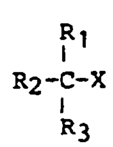

dans laquelle

- X représente un atome d'halogène, de préférence le chlore ou le brome.
- $R_1$, $R_2$, $R_3$, identiques ou différents, représentent un atome d'hydrogène ou un radical organique aliphatique, aromatique ou hétérocyclique, saturé ou insaturé, substitué ou non substitué, éventuellement substitué par au moins un groupement non électroréductible dans les conditions de l'électrolyse, ou bien encore $R_1$ et $R_2$ forment un cycle aliphatique.

4. Procédé selon la revendication 3 caractérisé en ce que X représente le chlore ou le brome, $R_1$ et $R_2$

l'hydrogène et $R_3$ soit un groupement hydrocarboné saturé ou insaturé, substitué ou non substitué soit un groupement aromatique.

5. Procédé selon l'une quelconque des revendications précédentes caractérisé en ce qu'on réduit un mélange de plusieurs, deux de préférence, halogénures organiques alpha saturés différents.

6. Procédé selon l'une quelconque des revendications 1 à 4 caractérisé en ce qu'on réduit un seul halogénure organique alpha saturé.

7. Procédé selon l'une quelconque des revendications précédentes caractérisé en ce que le catalyseur à base de nickel est obtenu par mélange d'un halogénure de nickel et d'un ligand organique bidentate biazoté, de préférence la 2,2'-bipyridine.

8. Procédé selon l'une quelconque des revendications précédentes caractérisé en ce que le catalyseur à base de nickel est obtenu en présence d'un coligand éthylénique, par exemple l'éthylène.

9. Procédé selon l'une quelconque des revendications précédentes caractérisé en ce qu'on ajoute l'halogénure organique alpha saturé ou un mélange d'halogénures organiques alpha saturés progressivement au cours de l'électrosynthèse.

## Ansprüche

1. Verfahren zur elektrochemischen Synthese von α-gesättigten Ketonen durch elektrochemische Reduktion von α-gesättigten organischen Halogeniden in einer mit Elektroden ausgestatteten Elektrolysezelle in einem organischen Lösungsmittel, das einen Trägerelektrolyten enthält, wobei die Anode aus einem Metall besteht, das unter den reduzierenden Metallen und ihren Legierungen ausgewählt ist, **dadurch gekennzeichnet,** daß die Reduktion in Gegenwart von $CO_2$ und eines Katalysators auf der Basis von Nickel, das mit einem zweizähnigen organischen Liganden mit zwei Stickstoffatomen komplexiert ist, durchgeführt wird.

2. Verfahren nach Anspruch 1, dadurch gekennzeichnet, daß die Anode aus einem Metall besteht, das unter Magnesium und seinen Legierungen ausgewählt ist.

3. Verfahren nach einem der vorhergehenden Ansprüche, dadurch gekennzeichnet, daß die α-gesättigten organischen Halogenide der allgemeinen Formel A

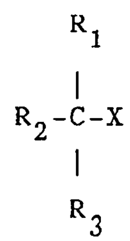

entsprechen, in der bedeuten

- X ein Halogenatom, vorzugsweise Chlor oder Brom, und
- $R_1$, $R_2$, $R_3$, die gleich oder verschieden sein können, ein Wasserstoffatom oder einen organischen aliphatischen, aromatischen oder heterocyclischen, gesättigten oder ungesättigten, substituierten oder nichtsubstituierten Rest, der gegebenenfalls mit mindestens einer unter den Bedingungen der Elektrolyse nicht reduzierbaren Gruppe substituiert ist, oder $R_1$ und $R_2$ zusammen einen aliphatischen Ring.

4. Verfahren nach Anspruch 3, dadurch gekennzeichnet, daß X Chlor oder Brom, $R_1$ und $R_2$ Wasserstoff und $R_3$ eine gesättigte oder ungesättigte, substituierte oder nichtsubstituierte Kohlenwasserstoffgruppe oder eine aromatische Gruppe bedeuten.

5. Verfahren nach einem der vorhergehenden Ansprüche, dadurch gekennzeichnet, daß ein Gemisch mehrerer, vorzugsweise zweier, verschiedener α-gesättigter organischer Halogenide reduziert wird.

6. Verfahren nach einem der Ansprüche 1 bis 4, dadurch gekennzeichnet, daß ein einziges α-gesättigtes organisches Halogenid reduziert wird.

7. Verfahren nach einem der vorhergehenden Ansprüche. dadurch gekennzeichnet, daß der Katalysator auf der Basis von Nickel durch Mischen eines Nickelhalogenids und eines zweizähnigen organischen Liganden mit zwei Stickstoffatomen, vorzugsweise 2,2'-Dipyridin, erhalten ist.

8. Verfahren nach einem der vorhergehenden Ansprüche, dadurch gekennzeichnet, daß der Katalysator auf der Basis von Nickel in Gegenwart eines ethylenischen Coliganden, z.B. von Ethylen, erhalten ist.

9. Verfahren nach einem der vorhergehenden Ansprüche, dadurch gekennzeichnet, daß das α-gesättigte organische Halogenid oder ein Gemisch α-gesättigter organischer Halogenide fortschreitend im Verlauf der

Elektrosynthese zugegeben wird.

## Claims

1. Process for the electrochemical synthesis of alpha-saturated ketones by electrochemical reduction of alpha-saturated organic halides in an electrolysis cell equipped with electrodes in an organic solvent medium containing a support electrolyte, the anode being made of a metal chosen from the group consisting of the reducing metals and their alloys, characterized in that the reduction takes place in the presence of $CO_2$ and of a catalyst based on nickel complexed with a bidentate organic ligand containing two nitrogen atoms.

2. Process according to Claim 1, characterized in that the anode is made of a metal chosen from the group consisting of magnesium and its alloys.

3. Process according to one of the preceding claims, characterized in that the alpha-saturated organic halides correspond to the general formula (A)

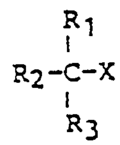

in which

X denotes a halogen atom, preferably chlorine or bromine, and

$R_1$, $R_2$ and $R_3$, which are identical or different, denote a hydrogen atom or a substituted or unsubstituted, saturated or unsaturated, aliphatic, aromatic or heterocyclic organic radical, optionally substituted by at least one group which cannot be electrically reduced under the conditions of the electrolysis, or else $R_1$ and $R_2$ form an aliphatic ring.

4. Process according to Claim 3, characterized in that X denotes chlorine or bromine, $R_1$ and $R_2$ hydrogen, and $R_3$ either a substituted or unsubstituted, saturated or unsaturated hydrocarbon group or an aromatic group.

5. Process according to any one of the preceding claims, characterized in that a mixture of several, preferably two, different alpha-saturated organic halides is reduced.

6. Process according to any one of Claims 1 to 4, characterized in that a single alpha-saturated organic halide is reduced.

7. Process according to any one of the preceding claims, characterized in that the nickel-based catalyst is obtained by mixing a nickel halide with a bidentate organic ligand containing two nitrogen atoms, preferably 2,2'-bipyridine.

8. Process according to any one of the preceding claims, characterized in that the nickel-based catalyst is obtained in the presence of an ethylenic coligand, for exemple ethylene.

9. Process according to any one of the preceding claims, characterized in that the alpha-saturated organic halide or a mixture of alpha-saturated organic halides is added gradually during the electrosynthesis.